# EUROPÄISCHE PATENTSCHRIFT

(11) **EP 3 600 507 B1**
(45) Veröffentlichungstag und Bekanntmachung des Hinweises auf die Patenterteilung: **28.07.2021**
(21) Anmeldenummer: 18716158.3
(22) Anmeldetag: 27.03.2018
(51) Int. Cl.: B29D 23/00, A61M 16/04

(54) **TRACHEALKANÜLE MIT EINER INTEGRIERTEN LEITUNG**
TRACHEAL CANNULA WITH AN INTEGRATED LINE
CANULE TRACHÉALE POURVUE D'UNE CONDUITE INTÉGRÉE

(30) Priorität: 29.03.2017 DE 102017106750
(43) Veröffentlichungstag der Anmeldung: 05.02.2020
(73) Patentinhaber: Tracoe Medical GmbH, 55268 Nieder-Olm (DE)
(72) Erfinder: SCHNELL, Ralf, 63500 Seligenstadt (DE); HAHN, Andreas, 55291 Saulheim (DE)
(74) Vertreter: WSL Patentanwälte Partnerschaft mbB
(86) Internationale Anmeldenummer: PCT/EP2018/057816
(87) Internationale Veröffentlichungsnummer: WO 2018/178098

(56) Entgegenhaltungen:
- US-A- 5 647 358

## Beschreibung

Die vorliegende Erfindung betrifft eine Trachealkanüle mit einer integrierten Leitung. Derartige Trachealkanülen auf welche sich die vorliegende Erfindung bezieht, insbesondere Tracheostomiekanülen oder Endotrachealtuben, haben ein Kanülenrohr aus Kunststoff, welches ein proximales Ende mit einer proximalen Öffnung, ein distales Ende mit einer distalen Öffnung und mindestens eine sich in axialer Richtung des Kanülenrohrs parallel zu diesem erstreckende, separate Leitung aufweist. Ein entsprechendes Beispiel zeigt die DE 197 34 821 A1.

Trachealkanülen werden in die Trachea eines Patienten eingeführt, um den Patienten mit Luft bzw. Atemgas zu versorgen. Das proximale Ende der Trachealkanüle ist außerhalb des Patienten mit einer Beatmungsvorrichtung verbindbar, während das distale Ende der Trachealkanüle in die Trachea einführbar ist. Das die zwei Enden verbindende Kanülenrohr dient mit seinem zentralen Lumen der Hindurchleitung von Atemgas.

Die separate Leitung ist beispielsweise für das Befüllen eines Cuffs mit Luft mit dem Cuff verbindbar oder auch zum Absaugen von Sekret nutzbar. Die Leitung soll das Lumen der Trachealkanüle möglichst nicht verkleinern, und ist deshalb oft unmittelbar auf die äußere Oberfläche des Kanülenrohres aufgebracht, beispielsweise mit dem Kanülenrohr verklebt oder verschweißt. Allerdings kann eine auf der Außenseite aufgebrachte Leitung ein Einführen der Trachealkanüle in die Trachea zusätzlich erschweren. Gemäß der DE 197 34 821 A1 derselben Anmelderin ist deshalb eine solche Leitung in die Wand des Kanülenrohrs integriert.

Die US 5,647,358 beschreibt eine inter vivo dehnbare Röhre, konkret eine Trachealkanüle, die in Längsrichtung geschlitzt ist, wobei der geschlitzte Bereich mindestens ein im Querschnitt in etwa H-förmiges Profilelement aufweist, welches bezüglich des Außenumfanges der Kanüle verjüngte Randabschnitte beiderseits des Schlitzes umgreift, wobei die verjüngte Randabschnitte beiderseits des Schlitzes in dem H-förmigen Profilelement in Umfangsrichtung verschiebbar sind.

Dabei wird zwischen dem zentralen Quersteg des H-förmigen Profils und dem diesem Quersteg zugewandten Rand der verjüngten Bereiche ein Dehnungsvolumen gebildet, welches durch eine im Inneren der Kanülenwand verlaufende Leitung befüllbar ist. Durch Befüllen der Leitung wird dieses Dehnungsvolumen gefüllt und drückt die beiderseits des Schlitzes in dem H-Profil aufgenommenen Randabschnitte des Kanülenrohres in Umfangsrichtung auseinander. Hierdurch weitet sich der Umfang und der Durchmesser der Trachealkanüle.

Trachealkanülen mit einem Kanülenrohr aus Kunststoff werden zumeist entweder mittels Extrusion- oder Spritzgussverfahren hergestellt. Kanülenrohre mit einem konischen Innen- und/oder Außendurchmesser und/oder mit einer vorgegebenen Krümmung können allerdings nicht mit der notwendigen Reproduzierbarkeit mittels eines Extrusionsverfahrens hergestellt werden. In einem Spitzgussverfahren können im Gegensatz zu einem Extrusionsverfahren keine Leitungen in die Wand des Kanülenrohrs eingeformt werden.

Vor diesem Hintergrund ist es eine Aufgabe der vorliegenden Erfindung eine einfach herstellbare Trachealkanüle mit mindestens einer integrierten Leitung bereitzustellen.

Erfindungsgemäß wird die Aufgabe durch eine Trachealkanüle gemäß der angehängtenAnsprüche definiert.

Die Erfindung betrifft darüber hinaus ein Verfahren zur Herstellung der erfindungsgemäßen Trachealkanüle, wobei zunächst ein Kanülenrohr und ein Profilelement als zwei getrennte Komponenten der Trachealkanüle bereitgestellt werden. Das Kanülenrohr ist aus einem weicheren Kunststoff hergestellt als das Profilelement und hat eine entlang mindestens eines Umfangsabschnittes rückspringende Außenwand mit einer entsprechend verringerten Wandstärke, wodurch eine Aussparung auf der Außenseite des Kanülenrohrs definiert ist (Vorzugsweise über seitliche Hinterschnitte (0,1-1 mm) gebildete Taschen, wie bei einem Clip-Verschluss). Nach deren Bereitstellung wird das Profilelement in der Aussparung auf der Außenseite des Kanülenrohrs kraft- und/oder formschlüssig angeordnet, sodass in dem Fügebereich zwischen dem Profilelement und dem Kanülenrohr ein Hohlraum bereitgestellt wird, der sich in axialer Richtung der Trachealkanüle erstreckt und die Leitung bildet. Anschließend werden das Profilelement und das Kanülenrohr dauerhaft miteinander verbunden, vorzugsweise durch Verkleben oder Verschweißen. Das erfindungsgemäße Verfahren gliedert sich demnach in die Schritte:
- Bereitstellen des Kanülenrohrs,
- Bereitstellen des mindestens einen Profilelements,
- kraft- und/oder formschlüssiges Anordnen des zumindest einen Profilelements in der Aussparung auf der Außenseite des Kanülenrohrs, und
- dichtes Verbinden des zumindest einen Profilelements mit der Außenfläche des Kanülenrohrs über die axiale Länge des Profilelementes hinweg.

Die die Aussparung definierenden Flächen werden dabei als Teil der Außenfläche des Kanülenrohrs angesehen.

Da das Profilelement aus einem härteren Kunststoff hergestellt ist als das Kanülenrohr, stabilisiert das Profilelement das Kanülenrohr und verhindert insbesondere, dass die in dem Fügebereich zwischen dem Profilelement und dem Kanülenrohr angeordnete Leitung oder das zentrale Lumen zum Hindurchführen von Atemgas durch das Kanülenrohr durch ein Verbiegen des Kanülenrohrs weitgehend verschlossen wird. Das Kanülenrohr selbst kann dann aus einem relativ weichen Kunststoff hergestellt werden, der ohne Versteifung durch das erfindungsgemäße Profilelement eher nicht verwendet werden würde. Beispielsweise weist in einer Ausführungsform der Kunststoff des Kanülenrohrs eine Shore-A-Härte in einem Bereich von 40 bis 90, vorzugsweise eine Shore-A-Härte in einem Bereich von 70 bis 80, und der Kunststoff des Profilelements vorzugsweise eine Shore-Härte in einem Bereich von A90 bis D70 auf.

Die in dem Fügebereich zwischen Profilelement und Kanülenrohr angeordnete Leitung dient der Hindurchleitung eines Fluids. Beispielsweise ist mit der Leitung ein Cuff verbindbar, wobei der Cuff durch ein durch die Leitung hindurchgeführtes Fluid befüllbar ist und der Cuff auch über die Leitung entleert werden kann. Es kann auch eine zweite Leitung parallel zu der ersten Leitung vorgesehen sein, wobei über die erste Leitung ein Cuff befüllbar und über die zweite Leitung ein Fluid abgesaugt und/oder ein Spülfluid zugeführt werden kann. Insbesondere können bei Bereitstellung von zwei oder mehr Leitungen eine Leitung für die Zufuhr eines Fluids und eine andere gleichzeitig zur Absaugung des Fluids verwendet werden. Des Weiteren ist auch eine Leitung zur Zuführung von Luft in den subglottischen Bereich denkbar, die den Patienten bei der Phonation unterstützt.

In einer Ausführungsform wird der den Innenquerschnitt der Leitung begrenzende und dem Kanülenrohr zugewandte Flächenabschnitt des Profilelementes durch eine Nut gebildet, die sich in Längsrichtung entlang des Profilelementes erstreckt, und/oder der den Innenquerschnitt der Leitung begrenzende und dem Profilelement zugewandte Flächenabschnitt des Kanülenrohrs ist als Nut ausgebildet. Vorzugsweise hat die Nut ein U-Profil. Sind beide den Innenquerschnitt der Leitung begrenzende Flächenabschnitte Nutabschnitte, so sind diese derart zueinander ausgerichtet, dass die zwei Nutabschnitte einen geschlossenen, fluiddichten Innenquerschnitt bilden. Weist nur einer der die Leitung begrenzenden Flächenabschnitte des Profilelementes oder des Kanülenrohrs eine Nut auf, deckt der entsprechend andere, nicht-nutförmige Flächenabschnitt die Nut ab. Auf diese Weise bildet der nicht-nutförmige und den Innenquerschnitt der Leitung begrenzende Flächenabschnitt eine Abdeckung für die Nut. Bei dieser Ausführungsform ist eine exakte Ausrichtung der Flächenabschnitte von Profilelement und Aussparung für den Erhalt einer dichten Leitung weniger kritisch. Herstellungsbedingte Abweichungen bei der Positionierung der Nut sind tolerierbar. Damit der Außenquerschnitt der Trachealkanüle klein und der Innendurchmesser des zentralen Lumens des Kanülenrohrs für die Hindurchleitung von Atemgas groß gehalten werden kann, ist erfindungsgemäß in dem Fügebereich zwischen dem Kanülenrohr und dem Profilelement zumindest eine weitere sich in axialer Richtung erstreckende Leitung angeordnet, die einen Innenquerschnitt aufweist, der durch einen dem Kanülenrohr zugewandten Flächenabschnitt des Profilelementes und einen dem Profilelement zugewandten Flächenabschnitt des Kanülenrohrs begrenzt ist. Der Innenquerschnitt der mindestens zwei Leitungen kann klein gehalten werden, wenn diese zusammen dem gleichen Verwendungszweck dienen. Beispielsweise können die mindestens zwei Leitungen zum Befüllen mit einem Cuff verbunden sein. Das Gesamtfördervolumen verteilt sich auf die Anzahl der verwendeten Leitungen, deren Einzelinnenquerschnitt entsprechend klein ausgeführt sein kann. Die zumindest zwei Leitungen können aber auch für unterschiedliche Zwecke verwendet werden. Beispielsweise können eine Leitung mit einem Cuff und eine weitere Leitung mit einer Absaugvorrichtung verbunden sein.

Vorzugsweise hat die eine Leitung oder haben die zumindest zwei oder mehreren Leitungen einen hydraulischen Durchmesser, der vorzugsweise ausgewählt ist aus den Bereichen 1 mm bis 4 mm oder 0,3 mm bis 1 mm. Eine Leitung mit einem hydraulischen Durchmesser in dem Bereich 1 mm bis 4 mm ist insbesondere für die Durchleitung von Flüssigkeit geeignet, während eine Leitung mit einem hydraulischen Durchmesser in dem Bereich von 0,3 mm bis 1 mm für die Durchleitung von Gas geeignet ist.

In einer Ausführungsform bilden die Außenfläche des Profilelements und die Außenfläche des Kanülenrohrs gemeinsam eine geschlossene Außenkontur, vorzugsweise eine Kreisform, oder eine elliptische Form, wobei das Profilelement in einer entsprechenden Aussparung an der Außenfläche des Kanülenrohrs angeordnet ist. Die gemeinsame, geschlossene Außenkontur weist keine Kanten, Ecken oder Vorsprünge auf, die die Trachea oder ein Tracheostoma beim Einführen der Trachealkanüle beschädigen könnten. Ein in einer Aussparung abschnittsweise aufgenommenes Profilelement ist teilweise in den Kunststoff des Kanülenrohrs eingebettet, sodass der Fügebereich zwischen dem Profilelement und dem Kanülenrohr im Wesentlichen innerhalb der einhüllenden Außenkontur des Kanülenrohres angeordnet ist. Der Fügebereich ist weitestgehend durch den ihn umgebenden Kunststoff geschützt.

Um eine Positionierung und Ausrichtung des Profilelementes relativ zu dem Kanülenrohr bei der Herstellung der Trachealkanüle zu vereinfachen, und um insbesondere ein nachträgliches Anbringen des Profilelementes ohne weitere Spannelemente zu ermöglichen, die das Profilelement in der ausgewählten Position und Ausrichtung relativ zu dem Kanülenrohr halten, sind in einer Ausführungsform das Profilelement und das Kanülenrohr formschlüssig und/oder kraftschlüssig miteinander verbunden, vorzugsweise miteinander verrastet. Das Profilelement und das Kanülenrohr weisen einander zugewandte Befestigungsmittel auf, die miteinander in Eingriff bringbar sind. Nach dem Bereitstellen des Profilelementes und des Kanülenrohrs, wird das Profilelement zunächst kraft- und/oder formschlüssig an dem Kanülenrohr befestigt. Die Befestigungsmittel des Kanülenrohrs und des Profilelementes werden miteinander in Eingriff gebracht, sodass das Profilelement an dem Kanülenrohr in einer durch die Befestigungsmittel vorgegebenen Position und Orientierung befestigt ist. Sind die Befestigungsmittel Rastmittel, weist also das Profilelement und das Kanülenrohr miteinander in Eingriff bringbare Rastnasen und Rastaufnahmen auf, kann das Profilelement mit dem Kanülenrohr verrastet werden. Anschließend kann das Profilelement an dem Kanülenrohr dauerhaft befestigt werden. Beispielsweise kann das Profilelement an dem Kanülenrohr angeschweißt, verklebt oder angespritzt werden. Durch das Anbringen des Profilelements an dem Kanülenrohr wird die zusätzliche Leitung abgedichtet.

In manchen Fällen ist es wünschenswert, eine Tracheostomiekanüle in Form einer Kombination aus einer Außen- und einer Innenkanüle zu verwenden. Um das Einführen einer Innenkanüle in das erfindungsgemäße Kanülenrohr zu vereinfachen, weist in einer Ausführungsform die Innenwand des Kanülenrohres einen aus einem härteren Kunststoff hergestellten sich in axialer Richtung des Kanülenrohrs erstreckenden Gleitabschnitt auf, der zu einem Teil in einer Aussparung der Wand des Kanülenrohres angeordnet ist und in das zentrale Lumen des Kanülenrohrs und, von den Innenwand desselben gemessen, um vorzugsweise 0,1 mm bis 0,5 mm, hineinragt. Der weichere Kunststoff des Kanülenrohres hat einen höheren Reibungskoeffizienten als der im Vergleich dazu härtere Kunststoff des Profilelements. Auf einem in das Lumen des Kanülenrohrs eingeführten Gleitabschnitt, der aus dem Kunststoff des Profilelements hergestellt ist, kann daher eine Innenkanüle oder ein Absaugschlauch leichter gleiten und durch das Kanülenrohr eingeführt werden. In einer Ausführungsform weist die Innenwand des Kanülenrohrs eine Mehrzahl von Gleitabschnitten, vorzugsweise zwei, drei oder vier Gleitabschnitte auf.

In weiterer Ausgestaltung dieser Ausführungsform weist das Kanülenrohr im Bereich der Aussparung einen schlitzförmigen Durchbruch auf, während das Profilelement an entsprechender Position einen sich in axialer Richtung des Kanülenrohrs erstreckenden Gleitabschnitt hat, der mit dem Profilabschnitt einstückig ausgebildet ist und durch einen Schlitz in der Wand des Kanülenrohrs hindurchgeführt ist und etwas in das zentrale Lumen des Kanülenrohrs, wiederum um vorzugsweise 0,1 mm bis 0,5 mm, hineinragt.

In einer weiteren Ausführungsform weist der in das zentrale Lumen des Kanülenrohrs hineinragende Gleitabschnitt eine Aufgleitschräge auf. Die Aufgleitschräge oder Fase verhindert, dass eine in das Kanülenrohr eingeführte Innenkanüle oder ein Absaugschlauch an der in das Lumen hineinragenden Gleitschiene beim Einführen hängenbleibt.

Damit die Trachealkanüle bei einer Untersuchung in einem in eine Trachea eingeführten Zustand bei der Verwendung bildgebender Verfahren gut sichtbar ist, weist in einer Ausführungsform das Kanülenrohr und/oder das Profilelement ein Kontrastmittel für ein bildgebendes Verfahren, vorzugsweise für die Röntgendiagnostik, Magnetresonanztomographie oder Sonografie, auf.

In einer Ausführungsform weist das Profilelement einen Konnektor zum Anschließen an eine Beatmungsvorrichtung auf, wobei vorzugsweise das Profilelement und der Konnektor einstückig aus demselben Kunststoff gefertigt sind. Der Konnektor dient dem Anschluss an eine Beatmungsvorrichtung und ist demnach an dem proximalen Ende des Kanülenrohrs zum Verbinden der proximalen Öffnung des Kanülenrohrs mit einer Beatmungsvorrichtung angeordnet. Vorzugsweise weist der Konnektor einen konischen Abschnitt auf, der abschnittsweise in die proximale Öffnung des Kanülenrohrs einsteckbar ist und mit der proximalen Öffnung dicht abschließt. Auf diese Weise kann der Konnektor in die proximale Öffnung nach Art einer Presspassung eingebracht werden. Der Konnektor kann in das Profilelement übergehen, welches sich von dem Konnektor in die Richtung des distalen Endes des Kanülenrohrs erstreckt. Die Presspassung des Konnektors unterstützt die Befestigung des Profilelementes an dem Kanülenrohr und umgekehrt.

In einer Ausführungsform weist das Kanülenrohr auf seiner Außenfläche ein zweites, sich in axialer Richtung erstreckendes Profilelement auf, wobei in dem Fügebereich zwischen dem Profilelement und dem Kanülenrohr zumindest eine weitere, sich in axialer Richtung erstreckende Leitung angeordnet ist, wobei das zweite Profilelement und/oder die zumindest eine weitere Leitung entsprechend dem zuvor beschriebenen Profilelement oder der Leitung ausgestaltet sind.

Vorzugsweise sind die Profilelemente auf gegenüberliegenden Seiten des Umfangs des Kanülenrohrs und spiegelbildlich bezüglich einer Ebene angeordnet, welche die Lumenachse enthält. Die aus einem im Vergleich zu dem Kunststoff des Kanülenrohres härteren Kunststoff gefertigten Profilelemente stabilisieren das Kanülenrohr.

Ist in einer Ausführungsform das Kanülenrohr in einer Ebene gebogen, verläuft eine zentrale Achse des Lumens der Kanüle um einen von 0° verschiedenen Winkel in einem in einer Ebene liegenden Bogen. Vorzugsweise beträgt der Biegewinkel etwa 90°. Bei einer solchen Ausführungsform sind zwei Profilelemente vorzugsweise auf diametral gegenüberliegenden Seiten des Kanülenrohrs und mit demselben Radius gekrümmt in zwei parallelen Ebenen beiderseits und spiegelbildlich bezüglich der durch den Krümmungsbogen der Achse des Lumens definierten Ebene angeordnet.

In einer Ausführungsform weisen die sich in axialer Richtung erstreckende Aussparung zur mindestens teilweisen Aufnahme eines passend geformten Profilelementes und das Profilelement komplementär zueinander angeordnete Vorsprünge und Ausnehmungen oder Vertiefungen auf. Vorzugsweise weist die Aussparung einen sich in axialer Richtung erstreckenden, vorspringenden Steg und das Profilelement eine sich entsprechend in axiale Richtung erstreckende Nut zur Aufnahme des Stegs auf. Die in der Aussparung angeordneten Vorsprünge des Kanülenrohrs sind, wie das Kanülenrohr selbst, aus einem im Vergleich zu dem Kunststoff des Profilelementes weicheren Kunststoff hergestellt. Ist das Kanülenrohr in einer Ebene gebogen, sind der sich axial erstreckende Steg und die sich axial erstreckende Ausnehmung oder Vertiefung in dem Bereich des Krümmungsbogens angeordnet. Der aus dem weicheren Kunststoff gefertigte Steg durchdringt das Profilelement in dem Bereich des Krümmungsbogens. Durch die sichere Verbindung mit dem Profilelement wird ein Abknicken des Kanülenrohres verhindert. Es versteht sich, dass der Vorsprung und die Ausnehmung oder Vertiefung andere geometrische Formen, wie beispielsweise Zylinder, Dreiecke, Quader, Rechtecke, aufweisen können. Weist die Trachealkanüle mehre Aussparungen und passende Profileelemente auf, so weisen vorzugsweise jede Aussparung einen Vorsprung und jedes Profilelement eine passende Ausnehmung oder Vertiefung auf.

Um die Flexibilität des Kanülenrohrs an bestimmten Abschnitten, wie zum Beispiel im Bereich eines Cuffs, zu erhöhen, ist in einer Ausführungsform vorgesehen, dass das Kanülenrohr lokal eine Mehrzahl von Ausnehmungen in der Kanülenwand aufweist. Vorzugsweise ist die Mehrzahl von Ausnehmungen in einem Umfangsabschnitt des Kanülenrohrs angeordnet, der von einem an dem Kanülenrohr angeordneten Cuff abgedeckt ist.

In einer Ausführungsform nehmen der Innenquerschnitt und/oder der Außenquerschnitt des Kanülenrohrs in Richtung des distalen Endes ab. Das Kanülenrohr weist somit eine konische Innen- und/oder Außenwand auf.

Das Kanülenrohr einer Ausführungsform weist ein angeformtes Kanülenschild auf, wobei vorzugsweise das Kanülenrohr und das Kanülenschild einstückig aus demselben Kunststoff hergestellt sind. Damit ein mit einem Konnektor verbundenes Profilelement mit der proximal zu dem Kanülenschild angeordneten Öffnung des Kanülenrohrs in Eingriff bringbar ist, und dennoch die Profilelemente sich in die Richtung des distalen Endes der Trachealkanüle erstrecken, weist das Kanülenschild in einer Ausführungsform eine Durchführung für das Profilelement auf.

Soweit in der vorliegenden Beschreibung, einschließlich der nachfolgenden Figurenbeschreibung Aspekte der Erfindung im Hinblick auf die Trachealkanüle beschrieben wurden, so gelten diese auch für das entsprechende Verfahren zum Herstellen einer solchen Trachealkanüle und umgekehrt.

Weitere Vorteile, Merkmale und Anwendungsmöglichkeiten der vorliegenden Erfindung werden anhand der vorliegenden Beschreibung einer Ausführungsform und der dazugehörigen Figuren deutlich, wobei gleiche Bezugszeichen auf gleiche Elemente verweisen. Es zeigen:
- Figur 1:: eine Perspektivansicht auf das Kanülenrohr einer erfindungsgemäßen Ausführungsform einer Trachealkanüle, dargestellt ohne Profilelemente,
- Figur 2:: eine Ansicht auf das proximale Ende des Kanülenrohrs gemäß der Ausführungsform aus Figur 1, dargestellt mit Profilelementen,
- Figur 3a:: eine perspektivische Detailansicht auf das proximale Ende des Kanülenrohrs einer Trachealkanüle gemäß der Ausführungsform aus Figur 2,
- Figur 3b: eine Draufsicht auf das proximale Ende des Kanülenrohrs einer Trachealkanüle gemäß der Ausführungsform aus Figur 3a,
- Figur 3c: einen Querschnitt durch das proximale Ende des Kanülenrohrs einer Trachealkanüle gemäß der der Ausführungsform aus Figur 3a,
- Figur 4:: eine teilweise weggebrochene Seitenansicht einer Trachealkanüle gemäß einer weiteren Ausführungsform der vorliegenden Erfindung,
- Figur 5:: eine Perspektivansicht auf das Kanülenrohr der Trachealkanüle aus Figur 4, dargestellt ohne Profilelemente und Cuff,
- Figur 6:: eine teilweise weggebrochene Seitenansicht einer Trachealkanüle gemäß einer weiteren Ausführungsform der vorliegenden Erfindung, und
- Figur 7:: ein Querschnitt durch die Trachealkanüle entlang der Schnittlinie A-A gemäß Figur 6.

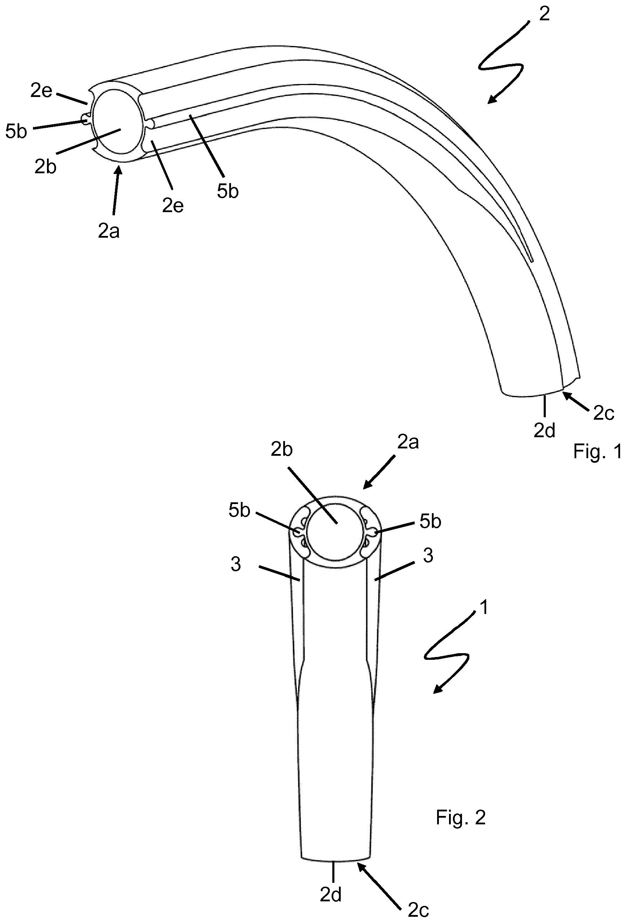

In der Figur 1 ist ein gebogenes Kanülenrohr 2 einer erfindungsgemäßen Trachealkanüle 1 gemäß einer ersten Ausführungsform ohne Profilelemente dargestellt. Das Kanülenrohr 2 ist aus Kunststoff gefertigt und weist ein proximales Ende 2a mit einer proximalen Öffnung 2b sowie ein distales Ende 2c mit einer distalen Öffnung 2d auf. Über einen Teil seiner Außenseite hat das Kanülenrohr 2 zwei rückspringende Bereiche mit reduzierter Wandstärke, die jeweils eine sich in axialer Richtung erstreckende Aussparung 2e definieren. Die Aussparungen 2e sind diametral auf gegenüberliegenden Seiten des Kanülenrohrs 2 angeordnet. Die Aussparungen 2e erstrecken sich von dem proximalen Ende 2a des Kanülenrohres 2 in axialer Richtung des Kanülenrohrs 2. Die Aussparungen 2e enden im Abstand vor dem distalen Ende 2b des Kanülenrohrs 2.

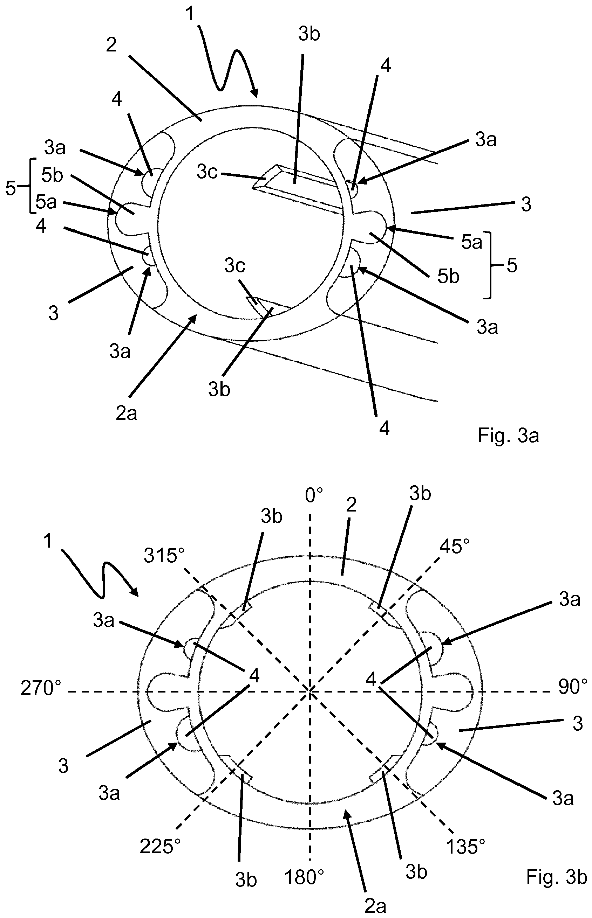

In jede der Aussparungen 2e ist ein passend geformtes Profilelement 3 einsetzbar, wie es in der Figur 2 und noch detaillierter in Figur 3a-c abschnittsweise dargestellt ist. Jedes Profilelement 3 hat zwei Nuten 3a, die sich in axialer Richtung der Trachealkanüle 1 erstrecken. In einem Zustand, in dem das Profilelement 3 in die Aussparung 2e des Kanülenrohrs 2 eingesetzt ist, sind die Nuten 3a zwischen dem Profilelement 3 und dem Kanülenrohr 2, genauer gesagt dem Grund der Aussparung(en), angeordnet. Die Nuten 3a sind dem Kanülenrohr 2 zugewandt und werden von einem dem jeweiligen Profilelement 3 zugewandten Flächenabschnitt des Kanülenrohrs 2 abgedeckt. Die Nuten 3a und die ihr jeweils zugewandten Flächen des Kanülenrohrs 2 begrenzen den Innenquerschnitt der separaten Leitungen 4, die folglich zwischen einander zugewandten Flächen des Profilelementes 3 und der Aussparung 2e gebildet werden. Zwei der insgesamt vier gezeigten Leitungen 4 haben einen größeren Innenquerschnitt als die übrigen zwei Leitungen 4. Die Leitungen 4 mit einem größeren hydraulischen Querschnitt werden vorzugsweise für die Durchleitung von Flüssigkeit und die Leitungen 4 mit einem kleineren Querschnitt werden vorzugsweise für die Durchleitung von Gas verwendet. Eine der Leitungen 4 ist mit einem Cuff verbindbar, sodass dieser mittels eines durch die Leitungen 4 zugeführten Fluids befüllbar ist.

Jedes der Profilelemente 3 ist in je einer der Aussparungen 2e mit Rastmitteln 5 verrastbar. Die Profilelemente 3 weisen eine Rastaufnahme 5a auf, in die ein an dem Kanülenrohr 2 in der Aussparung 2e angeordnete Rastnase 5b eingreift. Nach dem Anbringen der Profilelemente 3 in den Aussparungen 2e werden die Profilelemente 3 an das Kanülenrohr 2 geklebt. Durch das Verkleben sind die Profilelemente 3 nicht nur dauerhaft mit dem Kanülenrohr 2 verbunden, sondern es werden dadurch auch die Leitungen 4 zusätzlich abgedichtet.

Das Kanülenrohr 2 und die Profilelemente 3 sind aus Kunststoff hergestellt, wobei der Kunststoff des Kanülenrohrs 2 weicher als der Kunststoff der Profilelemente 3 ist. In die Aussparungen 2e des Kanülenrohrs 2 eingesetzt, stabilisieren die Profilelemente 3 die Trachealkanüle 1 und verhindern ein ungewolltes Abknicken des relativ weichen Kanülenrohrs 2, was ansonsten die Leitungen 4 oder das zentrale Lumen zum Hindurchführen von Atemgas blockieren könnte. Die Aussparungen 2e und die Profilelemente 3 sind daher auf gegenüberliegenden Seiten des Kanülenrohrs 2 spiegelbildlich bezüglich der durch den Krümmungsbogen der zentralen Achse des Lumens definierten Ebene angeordnet.

Die Profilelemente 3 und die Aussparungen 2e des Kanülenrohrs 2 sind derart ausgestaltet, dass die in die Aussparungen 2e eingesetzten Profilelemente 3 mit der Außenfläche des Kanülenrohrs 2 abschließen und eine im Wesentlichen geschlossene Außenkontur bilden. Die Profilelemente 3 haben eine zylindrische Außenfläche, die zusammen mit der Außenfläche des Kanülenrohrs 2c eine kreisförmige Außenkontur der Trachealkanüle 1 bilden.

In der Figur 3a ist das proximale Ende 2a der Trachealkanüle 1 aus den Figuren 1 und 2 in einer perspektivischen Detailansicht gezeigt. Die in die Aussparungen 2e des Kanülenrohrs 2 eingesetzten Profilelemente 3 und die Außenfläche des Kanülenrohrs 2 bilden gemeinsam eine elliptische Außenkontur.

Die Innenwand des Kanülenrohres 2 weist mindestens einen (oder vorzugsweise mehrere diametral gegenüberliegend angeordnete) Gleitabschnitt 3b auf, der sich in axialer Richtung erstreckt und teilweise in einer schlitz- oder nutartigen Wandaussparung des Kanülenrohres 2 aufgenommen ist und sich teilweise in das zentrale Lumen des Kanülenrohrs 2 erstreckt und um 0,1 mm bis 0,5 mm, gemessen von der Innenwand des Kanülenrohrs 2, in dieses hineinragt. In einer hier nicht dargestellten Variante ist der Gleitabschnitt einstückig mit dem Profilelement ausgebildet und erstreckt sich durch eine sich in axialer Richtung des Kanülenrohrs 2 erstreckende, schlitzförmige Durchbrechung. Damit eine Innenkanüle leichter in das zentrale Lumen des Kanülenrohrs 2 eingeführt werden und auf dem Gleitabschnitt 3b gleiten kann, weisen die vorzugsweise auf diametral gegenüberliegenden Seiten verlaufenden Gleitabschnitte 3b an ihren Enden je eine Aufgleitschräge 3c auf. In einer Draufsicht auf den Querschnitt der Trachealkanüle 1, wie er in der Figur 3b dargestellt ist, befinden sich die gedachten Mittelinien der auf gegenüberliegenden Seiten des Kanülenrohrs 2 angeordneten Profilelemente 3 auf 90° bis 270°. Die insgesamt vier Gleitabschnitte 3b der zwei Profilelemente 3 sind bei 45°, 135°, 225° und 315° angeordnet.

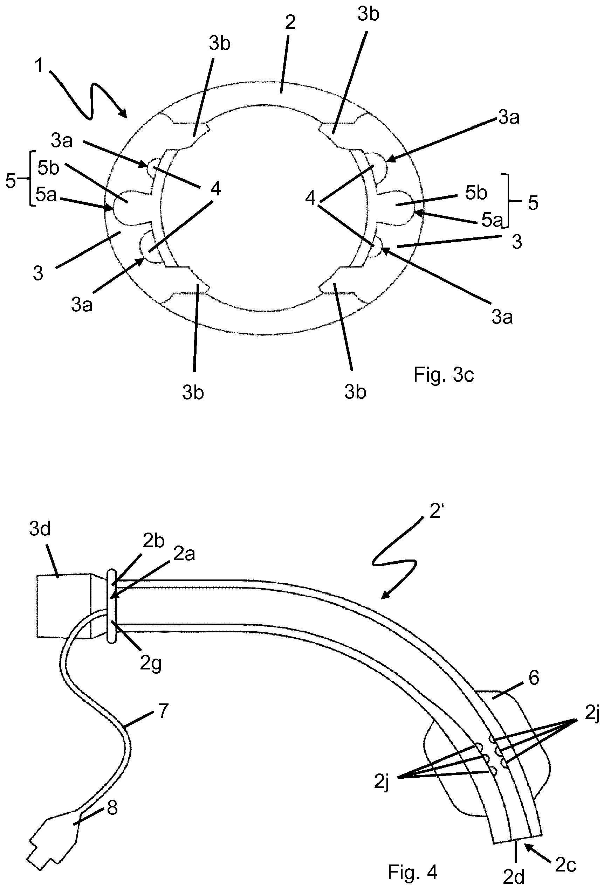

In der Figur 3c, die einen Querschnitt durch das proximale Ende 2a der Trachealkanüle 1 aus den Figuren 3a und 3b zeigt, sind die sich durch die Kanülenwand hindurcherstreckenden Gleitabschnitte 3b der in den Aussparungen 2e angeordneten Profilelemente 3 erkennbar.

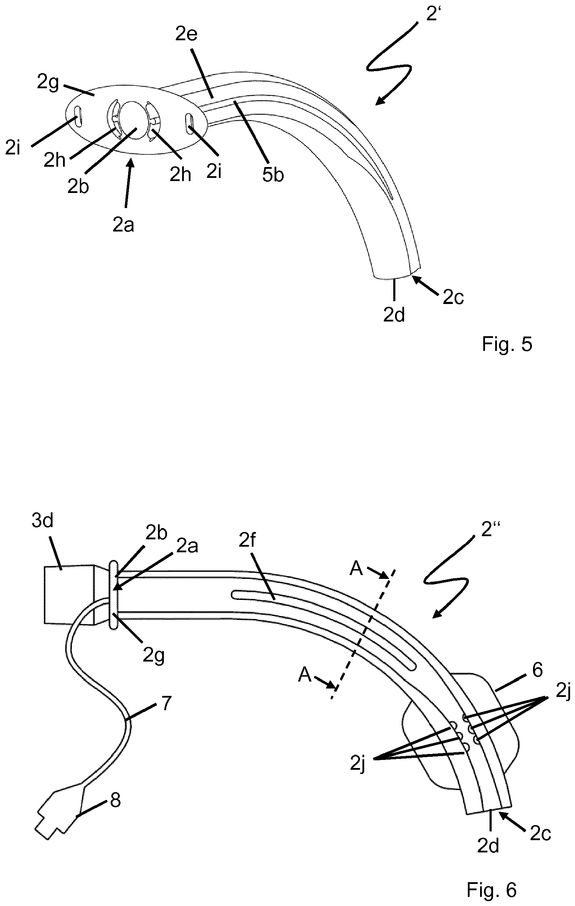

In der Figur 4 ist eine weitere Ausführungsform einer erfindungsgemäßen Trachealkanüle 1 gezeigt, wobei in der Figur 5 das dazugehörige Kanülenrohr 2' ohne Profilelemente 3 dargestellt ist. Das Kanülenrohr 2' weist zwei über einen Teil seines Umfangs auf seiner Außenseite im Querschnitt rückspringende Bereiche mit reduzierter Wandstärke auf, die jeweils eine sich in axialer Richtung erstreckende Aussparung 2e definieren. Die Profilelemente 3 und die Aussparungen 2c des Kanülenrohrs 2' erstrecken sich bei dieser Ausführungsform von dem proximalen Ende 2a bis zu dem distalen Ende 2c des Kanülenrohrs 2'. Das Kanülenrohr 2' hat einen konischen, in Richtung des distalen Ende 2c zulaufenden Außenquerschnitt.

Das proximale Ende 2a des Kanülenrohrs 2' weist ein Kanülenschild 2g auf, welches zusammen mit den Kanülenrohr 2' als ein Stück geformt ist. Das Kanülenrohr 2' und das Kanülenschild 2g sind aus einem weicheren Kunststoff gefertigt als die Profilelemente 3, die im Vergleich dazu aus einem härteren Kunststoff hergestellt sind.

Das Kanülenschild 2g weist zusätzlich zwei Durchbrechungen 2i für die Befestigung eines Kanülenbands sowie zwei seitliche Durchbrechungen 2h auf, die seitlich der proximalen Öffnung 2b des Kanülenrohrs 2 angeordnet sind. Durch jede der seitlichen Durchbrechungen 2h ist aus proximaler Richtung ein Profilelement 3 derart hindurchführbar, dass die hindurchgeführten Profilelemente 3 in die sich an die Durchbrechungen 2h anschließenden Aussparungen 2e des Kanülenrohrs 2 einbringbar sind.

In einem Zustand in dem die Profilelemente 3 in die Aussparungen 2e des Kanülenrohrs 2 eingebracht sind, steht ein Konnektor 3d, welcher zusammen mit den Profilelementen 3 als ein Stück gefertigt ist, in Eingriff mit der proximalen Öffnung 2b des Kanülenrohrs 2.

Die Aussparungen 2e weisen eine Rastnase 5b auf, die sich in axialer Richtung des Kanülenrohrs 2' erstreckt. Die Rastnase 5b ist mit einer Rastnasenaufnahme 5a in Eingriff bringbar, welche auf der Innenseite der Profilelemente 3 angeordnet ist. Links und rechts neben der Rastnasenaufnahme 5a weisen die Profilelemente 3 je eine Nut 3a auf, die sich in axialer Richtung des Profilelementes 3 erstrecken. Sind die Profilelemente 3 in den Aussparungen 2e des Kanülenrohrs 2' befestigt, begrenzen die Nuten 3a zusammen mit der dem jeweiligen Profilelement 3 zugewandten Fläche des Kanülenrohr 2' den Innenquerschnitt einer Leitung 4. Eine bzw. auch mehrere der separaten Leitungen 4 dienen dem Absaugen von Sekret, wobei die Profilelemente 3 hierzu Öffnungen aufweisen, die mit den Leitungen 4 verbunden sind. Eine oder weitere Leitungen 4 sind mit einem Cuff 6 und über einen Füllschlauch 7 mit einem Füllventil 8 verbunden. Mit dem Füllventil 8 kann über den Füllschlauch 7 und der Leitung 4 der Cuff 6 mit einem Fluid beaufschlagt oder ein Fluid aus dem Cuff 6 ausgeleitet werden.

In dem von dem Cuff 6 abgedeckten Abschnitt, weist das Kanülenrohr 2' eine Mehrzahl von in Umfangsrichtung verteilt angeordneten Ausnehmungen 2j auf. Die Ausnehmungen 2j erhöhen die Flexibilität des Kanülenrohrs 2'.

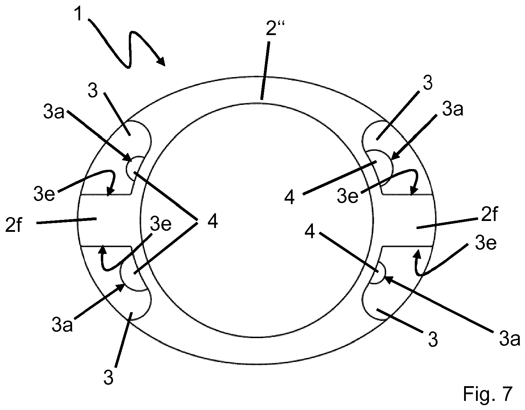

Die Figuren 6 und 7 zeigen eine weitere Ausführungsform einer Trachealkanüle 1 gemäß der vorliegenden Erfindung, wobei die Figur 6 eine teilweise weggebrochene Seitenansicht und die Figur 7 einen Querschnitt entlang der in der Figur 6 gezeigten Schnittlinie A-A zeigen. Die in den Figuren 6 und 7 gezeigte Ausführungsform unterscheidet sich von den Ausführungsformen der Figuren 1 bis 5 in der Ausgestaltung des Profilelementes und dessen Verbindung mit dem Kanülenrohr 2".

Das Kanülenrohr 2" der Trachealkanüle 1" ist aus Kunststoff gefertigt und weist ein proximales Ende 2a mit einer proximalen Öffnung 2b, ein distales Ende 2c mit einer distalen Öffnung 2d sowie vier sich in axialer Richtung des Kanülenrohrs 2" erstreckende separate Leitungen 4 auf. Je zwei der vier Leitungen 4 sind zwischen den einander zugewandten Flächen eines Profilelementes 3 und einer Aussparung 2e in dem Kanülenrohr 2" gebildet. Die zwei Profilelemente 3 sind diametral auf gegenüberliegenden Seiten des Kanülenrohrs 2" angeordnet und erstrecken sich in axialer Richtung des Kanülenrohrs 2". Das Kanülenrohr 2" ist gebogen, wobei in dem Bereich des Krümmungsbogens zwei durch einen Steg 2f getrennte Aussparungen 2e zur Aufnahme jeweils eines Zweiges der im Bereich des Steges 2f aufgetrennten Profilelemente 3 . Entsprechend hat jedes der Profilelemente 3 eine komplementär ausgestaltete Ausnehmung 3e zur Aufnahme des Steges 2f.

Der Steg 2f ist wie das Kanülenrohr 2" aus einem im Vergleich zu den Profilelementen 3 weicheren Kunststoff gefertigt. Auf diese Weise erhöhen die Stege die Flexibilität des Kanülenrohrs 2" in dem Krümmungsbereich, während die Profilelemente 3 nach wie vor ausreichend verhindern, dass das Kanülenrohr 2" sich durch Abknicken verengt.

Wie die Ausführungsform gemäß Figur 4, ist auch bei der Ausführungsform gemäß den Figuren 6 und 7 eine Mehrzahl von Ausnehmungen 2j in einem Umfangsabschnitt des Kanülenrohrs 2" angeordnet, der von einem an dem Kanülenrohr angeordneten Cuff 6 abgedeckt ist.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: Trachealkanüle
- 2, 2', 2": Kanülenrohr
- 2a: proximales Ende
- 2b: proximale Öffnung
- 2c: distales Ende
- 2d: distale Öffnung
- 2e: Aussparung
- 2f: stegförmiger Vorsprung
- 2g: Kanülenschild
- 2h: seitliche Durchbrechungen
- 2i: Durchbrechungen für die Befestigung eines Kanülenbands
- 2j: Ausnehmungen
- 3: Profilelement
- 3a: Nut
- 3b: Gleitabschnitt
- 3c: Aufgleitschräge
- 3d: Konnektor
- 3e: Ausnehmung oder Vertiefung
- 4: Leitung
- 5: Befestigungsmittel
- 5a: Rastnasenaufnahme
- 5b: Rastnase
- 6: Blockungsmanschette (Cuff)
- 7: Füllschlauch
- 8: Füllventil

## Patentansprüche

1. Trachealkanüle (1), insbesondere Tracheostomiekanüle oder Endotrachealtubus, mit einem Kanülenrohr (2) aus Kunststoff, welches ein proximales Ende (2a) mit einer proximalen Öffnung (2b), ein distales Ende (2c) mit einer distalen Öffnung (2d) und mindestens eine sich in axialer Richtung des Kanülenrohrs (2) erstreckende separate Leitung (4) aufweist, und einem Profilelement (3), das aus einem im Vergleich zu dem Kunststoff des Kanülenrohrs (2) härteren Kunststoff hergestellt ist **dadurch gekennzeichnet, dass** das Kanülenrohr (2) über einen Teil seines Umfangs auf seiner Außenseite einen im Querschnitt rückspringenden Bereich mit reduzierter Wandstärke aufweist, wodurch eine sich in axialer Richtung erstreckende Aussparung (2e) auf der Außenseite des Kanülenrohrs zur mindestens teilweisen Aufnahme des passend geformten Profilelementes (3) definiert wird, wobei die Leitung (4) zwischen den einander zugewandten Flächen des Profilelementes (3) und der Aussparung dadurch gebildet wird, dass deren Innenquerschnitt durch einen dem Kanülenrohr zugewandten Flächenabschnitt des Profilelementes (3) und einen dem Profilelement zugewandten Flächenabschnitt des Kanülenrohrs begrenzt ist.

2. Trachealkanüle (1) nach Anspruch 1, **dadurch gekennzeichnet, dass** der den Innenquerschnitt der Leitung (4) begrenzende und dem Kanülenrohr (2) zugewandte Flächenabschnitt des Profilelementes (3) durch eine Nut (3a) gebildet ist, die sich in Längsrichtung entlang des Profilelementes (3) erstreckt, und/oder der den Innenquerschnitt der Leitung (4) begrenzende und dem Profilelement (3) zugewandte Flächenabschnitt des Kanülenrohrs (2) ist als Nut (2f) ausgebildet.

3. Trachealkanüle (1) nach Anspruch 1 oder 2, **dadurch gekennzeichnet, dass** zwischen dem Kanülenrohr (2) und dem Profilelement (3) zumindest eine weitere sich in axialer Richtung des Kanülenrohrs (2) erstreckende Leitung (4) ausgebildet ist.

4. Trachealkanüle (1) nach einem der Ansprüche 1 bis 3, **dadurch gekennzeichnet, dass** die mindestens eine Leitung (4) einen hydraulischen Durchmesser im Bereich von 0,3 mm bis 1 mm und/oder dass im Falle mehrerer Leitungen (4) mindestens eine dieser Leitungen einen hydraulischen Durchmesser im Bereich von 1 mm bis 4 mm aufweist.

5. Trachealkanüle (1) nach einem der Ansprüche 1 bis 4, **dadurch gekennzeichnet, dass** die Außenfläche des Profilelements (3) zusammen mit der Außenfläche des Kanülenrohrs (2) eine im wesentlichen geschlossene Außenkontur, vorzugsweise eine Kreisform, Ellipsenform, bildet, wobei das Profilelement (3) in einer entsprechenden Aussparung (2e) in der Außenfläche des Kanülenrohrs (2) angeordnet ist.

6. Trachealkanüle (1) nach einem der Ansprüche 1 bis 5, **dadurch gekennzeichnet, dass** das Profilelement (3) und das Kanülenrohr (2) formschlüssig und/oder kraftschlüssig miteinander verbunden, vorzugsweise miteinander verrastet, sind

7. Trachealkanüle (1) nach einem der Ansprüche 1 bis 6, **dadurch gekennzeichnet, dass** der Kunststoff des Kanülenrohrs (2) eine Shore-A-Härte in einem Bereich von 40 bis 90, vorzugsweise eine Shore-A-Härte in einem Bereich von 70 bis 80, und der Kunststoff des Profilelements (3) vorzugsweise eine Shore-Härte in einem Bereich von A90 bis D70 aufweist.

8. Trachealkanüle (1) nach einem der Ansprüche 1 bis 7, **dadurch gekennzeichnet, dass** an der Innenwand des Kanülenrohres ein aus einem härteren Kunststoff hergestellter sich in axialer Richtung des Kanülenrohrs (2) erstreckender Gleitabschnitt (3b) vorgesehen ist, der zu einem Teil in einer Aussparung in der Wand des Kanülenrohres angeordnet ist und in das zentrale Lumen des Kanülenrohrs (2) und von der Innenwand desselben gemessen um vorzugsweise 0,1 mm bis 0,5 mm, hineinragt

9. Trachealkanüle (1) nach Anspruch 8 **dadurch gekennzeichnet, dass** der Gleitabschnitt (3b) aus einem Stück mit dem Profilelement (3) hergestellt ist und durch eine sich über einen Teil der Länge des Kanülenrohres (2) erstreckende, schlitzförmige Durchbrechung desselben(2) in das zentrale Lumen des Kanülenrohrs (2) hineinragt.

10. Trachealkanüle (1) nach Anspruch 8 oder 9, **dadurch gekennzeichnet, dass** der in das zentrale Lumen des Kanülenrohrs (2) hineinragende Gleitabschnitt (3b) eine Aufgleitschräge (3c) aufweist.

11. Trachealkanüle (1) nach einem der Ansprüche 1 bis 10, **dadurch gekennzeichnet, dass** das Kanülenrohr (2) und/oder das Profilelement (3) ein Kontrastelement für ein bildgebendes Verfahren, vorzugsweise für die Röntgendiagnostik, Magnetresonanztomographie oder Sonografie, aufweist.

12. Trachealkanüle (1) nach einem der Ansprüche 1 bis 11, **dadurch gekennzeichnet, dass** das Profilelement (3) einen Konnektor (3d) zum Anschließen einer Beatmungsvorrichtung aufweist, wobei vorzugsweise das Profilelement (3) und der Konnektor (3c) einstückig aus demselben Kunststoff gefertigt sind.

13. Trachealkanüle (1) nach einem der Ansprüche 1 bis 12, **dadurch gekennzeichnet, dass** das Kanülenrohr (2) auf seiner Außenfläche eine zweite Aussparung mit einem zweiten, sich in axialer Richtung erstreckenden Profilelement (3) aufweist, wobei zwischen dem Profilelement (3) und dem Kanülenrohr (2) zumindest eine weitere, sich axial erstreckende Leitung (4) ausgebildet ist, wobei das zweite Profilelement (2) und/oder die weitere Leitung (4) gemäß einem der vorgenannten Ansprüche ausgestaltet sind.

14. Trachealkanüle (1) nach einem der Ansprüche 1 bis 13, **dadurch gekennzeichnet, dass** die sich in axiale Richtung erstreckende Aussparung (2e) einen Vorsprung, vorzugsweise einen sich axial erstreckenden Steg (2f)" und das in die Aussparung eingesetzte Profilelement (3) eine komplementär zu dem Vorsprung ausgestaltete Ausnehmung (3e) aufweist.

15. Trachealkanüle (1) nach einem der Ansprüche 1 bis 14, **dadurch gekennzeichnet, dass** die Innen- und/oder Außenwand des Kanülenrohrs (2) sich in axialer Richtung vom proximalen zum distalen Ende hin konisch verjüngt.

16. Trachealkanüle (1) nach einem der Ansprüche 1 bis 15, **dadurch gekennzeichnet, dass** das Kanülenrohr (2) ein angeformtes Kanülenschild (2g) aufweist, wobei vorzugsweise das Kanülenschild (2g) und das Kanülenrohr (2) einstückig aus demselben Kunststoff hergestellt sind.

17. Verfahren zum Herstellen der Trachealkanüle (1) nach einem der Ansprüche 1 bis 16 mit den Schritten
- Bereitstellen eines Kanülenrohrs (2), mit einer entlang mindestens eines Umfangsabschnittes rückspringenden Außenwand und entsprechend verringerter Wandstärke, wodurch eine sich in axialer Richtung erstreckende Aussparung auf der Außenseite des Kanülenrohres definiert wird
- Bereitstellen des mindestens einen Profilelements (3),
- kraft- und/oder formschlüssiges Anordnen des zumindest einen Profilelements (3) in der Aussparung auf der Außenseite des Kanülenrohrs (2), und
- dichtes Verbinden des zumindest einen Profilelements (3) mit der Außenfläche des Kanülenrohrs (2) über die axiale Länge des Profilelementes hinweg.

## Claims

1. A tracheal cannula (1), in particular a tracheostomy cannula or endotracheal tube, comprising a cannula tube (2) of plastic which has a proximal end (2a) with a proximal opening (2b), a distal end (2c) with a distal opening (2d) and at least one separate line (4) extending in the axial direction of the cannula tube (2), and a profile element (3) made of a harder plastic compared to the plastic of the cannula tube (2), **characterised in that** the cannula tube (2) over part of its periphery on its outside has a region which is set back in cross section with a reduced wall thickness, whereby a recess (2a) which extends in the axial direction is defined on the outside of the cannula tube for at least partially receiving the matchingly shaped profile element (3), wherein the line (4) is formed between mutually facing surfaces of the profile element (3) and the recess by the internal cross-section-thereof being delimited by a surface portion of the profile element (3), that faces towards the cannula tube, and a surface portion of the cannula tube, that faces towards the profile element

2. A tracheal cannula (1) according to claim 1 **characterised in that** the surface portion of the profile element (3), that delimits the internal cross section of the line (4) and faces the cannula tube (2), is formed by a groove (3a) which extends in the longitudinal direction along the profile element (3) and/or the surface portion of the cannula tube (2), that delimits the internal cross section of the line (4) and faces the profile element (3), is in the form of a groove (2f).

3. A tracheal cannula (1) according to claim 1 or claim 2 **characterised in that** at least one further line (4) extending in the axial direction of the cannula tube (2) is provided between the cannula tube (2) and the profile element (3).

4. A tracheal cannula (1) according to one of claims 1 to 3 **characterised in that** the at least one line (4) is of a hydraulic diameter in the range from 0.3 mm to 1 mm and/or in the case of a plurality of lines (4) at least one of those Lines is of a hydraulic diameter in the range from 1 mm to 4 mm.

5. A tracheal cannula (1) according to one of claims 1 to 4 **characterised in that** the outside surface of the profile element (3) together with the outside surface of the cannula tube (2) forms a substantially closed outside contour, preferably a circular or elliptical shape, the profile element (3) being arranged in a corresponding recess (2e) in the outside surface of the cannula tube (2).

6. A tracheal cannula (1) according to one of claims 1 to 5 **characterised in that** the profile element (3) and the cannula tube (2) are connected together in positively locking and/or force-locking relationshippositively, preferably being latched to one another

7. A tracheal cannula (1) according to one of claims 1 to 6 **characterised in that** the plastic of the cannula tube (2) has a Shore A hardness in a range from 40 to 90, preferably a Shore A hardness in a range from 70 to 80, and the plastic of the profile element (3) preferably has a Shore hardness in a range from A90 to D70.

8. A tracheal cannula (1) according to one of claims 1 to 7 **characterised in that** provided on the inner wall of the cannula tube is a sliding portion (3b) made of a harder plastic and extending in the axial direction of the cannula tube (2), which is arranged partly in a recess in the wall of the cannula tube and projects into the central lumen of the cannula tube (2) and by preferably 0.1 mm to 0.5 mm as measured from the inner wall thereof.

9. A tracheal cannula (1) according to claim 8 **characterised in that** the sliding portion (3b) is in one piece with the profile element (3) and projects through a slit-shaped opening in the cannula tube (2) extending over a part of the length of the cannula tube (2) into the central lumen of the cannula tube (2).

10. A tracheal cannula (1) according to claim 8 or claim 9 **characterised in that** the sliding portion (3b) projecting into the central lumen of the cannula tube (2) has an inclined slide-on portion (3c).

11. A tracheal cannula (1) according to one of claims 1 to 10 **characterised in that** the cannula tube (2) and/or the profile element (3) has a contrast element for an imaging method, preferably for X-ray diagnostics, magnetic resonance tomography or sonography.

12. A tracheal cannula (1) according to one of claims 1 to 11 **characterised in that** the profile element (3) has a connector (3d) for connecting a ventilation device, wherein the profile element (3) and the connector (3c) are preferably made in one piece from the same plastic.

13. A tracheal cannula (1) according to one of claims 1 to 12 **characterised in that** the cannula tube (2) has on its outer surface a second recess with a second axially extending profile element (3), wherein at least one further axially extending line (4) is provided between the profile element (3) and the cannula tube (2), wherein the second profile element (2) and/or the further line (4) are of a configuration according to one of the preceding claims.

14. A tracheal cannula (1) according to one of claims 1 to 13 **characterised in that** the recess (2e) extending in the axial direction has a projection, preferably an axially extending web (2f), and the profile element (3) inserted into the recess has a recess (3e) of a complementary configuration to the projection.

15. A tracheal cannula (1) according to one of claims 1 to 14 **characterised in that** the inner and/or outer wall of the cannula tube (2) tapers conically in the axial direction from the proximal to the distal end.

16. A tracheal cannula (1) according to one of claims 1 to 15 **characterised in that** the cannula tube (2) has a cannula shield (2g) formed thereon, wherein the cannula shield (2g) and the cannula tube (2) are preferably made in one piece from the same plastic.

17. A method of producing a tracheal cannula (1) according to one of claims 1 to 16 comprising the steps
- providing a cannula tube (2) having an outer wall which is set back along at least a peripheral portion and a correspondingly reduced wall thickness, whereby a recess extending in the axial direction is defined on the outside of the cannula tube
- providing the at least one profile element (3),
- arranging the at least one profile element (3) in positively locking and/or force-locking relationship in the recess on the outside of the cannula tube (2), and
- sealingly connecting the at least one profile element (3) to the outer surface of the cannula tube (2) over the axial length of the profile element.

## Revendications

1. Canule trachéale (1), en particulier canule de trachéostomie ou tube endotrachéal, avec un tube de canule (2) en matière plastique présentant une extrémité proximale (2a) munie d'une ouverture proximale (2b), une extrémité distale (2c) munie d'une ouverture distale (2d) et au moins un conduit (4) séparé s'étendant dans la direction axiale du tube de canule (2), et un élément profilé (3) réalisé à partir d'une matière plastique qui est plus dure que la matière plastique du tube de canule (2), **caractérisée en ce que** le tube de canule (2) présente, sur une partie de sa circonférence et sur sa face extérieure, une région d'épaisseur de paroi réduite située en retrait en coupe transversale, de sorte qu'un évidement (2e) s'étendant dans la direction axiale est défini sur la face extérieure du tube de canule afin d'accueillir au moins partiellement l'élément profilé (3) de forme appropriée, dans laquelle le conduit (4) est formé entre les surfaces, orientées l'une vers l'autre, de l'élément profilé (3) et l'évidement de sorte que sa section transversale intérieure est limitée par une partie de surface, tournée vers le tube de canule, de l'élément profilé (3) et une partie de surface, tournée vers l'élément profilé, du tube de canule.

2. Canule trachéale (1) selon la revendication 1, **caractérisée en ce que** la partie de surface, délimitant la section transversale intérieure du conduit (4) et tournée vers le tube de canule (2), de l'élément profilé (3) est formée par une rainure (3a) qui s'étend dans la direction longitudinale le long de l'élément profilé (3), et/ou la partie de surface, délimitant la section transversale intérieure du conduit (4) et tournée vers l'élément profilé (3), du tube de canule (2) est réalisée sous forme de rainure (2f).

3. Canule trachéale (1) selon la revendication 1 ou 2, **caractérisée en ce qu'**au moins un autre conduit (4) s'étendant dans la direction axiale du tube de canule (2) est réalisé entre le tube de canule (2) et l'élément profilé (3).

4. Canule trachéale (1) selon l'une quelconque des revendications 1 à 3, **caractérisée en ce que** le au moins un conduit (4) présente un diamètre hydraulique situé dans la plage comprise entre 0,3 mm et 1 mm et/ou **en ce que**, lorsqu'il y a plusieurs conduits (4), au moins un desdits conduits présente un diamètre hydraulique situé dans la plage comprise entre 1 mm et 4 mm.

5. Canule trachéale (1) selon l'une quelconque des revendications 1 à 4, **caractérisée en ce que** la surface extérieure de l'élément profilé (3) forme avec la surface extérieure du tube de canule (2) un contour extérieur essentiellement fermé, de manière préférée de forme circulaire ou elliptique, dans laquelle l'élément profilé (3) est agencé dans un évidement (2e) correspondant présent au sein de la surface extérieure du tube de canule (2).

6. Canule trachéale (1) selon l'une quelconque des revendications 1 à 5, **caractérisée en ce que** l'élément profilé (3) et le tube de canule (2) sont reliés l'un à l'autre par complémentarité de forme et/ou de force, de manière préférée par encliquetage.

7. Canule trachéale (1) selon l'une quelconque des revendications 1 à 6, **caractérisée en ce que** la matière plastique du tube de canule (2) présente une dureté Shore A située dans une plage comprise entre 40 et 90, de manière préférée une dureté Shore A située dans une plage comprise entre 70 et 80, et la matière plastique de l'élément profilé (3) présente de manière préférée une dureté Shore située dans une plage comprise entre A90 et D70.

8. Canule trachéale (1) selon l'une quelconque des revendications 1 à 7, **caractérisée en ce qu'**une partie de glissement (3b) réalisée en une matière plastique plus dure, s'étendant dans la direction axiale du tube de canule (2), agencée en partie dans un évidement au sein de la paroi du tube de canule et faisant saillie dans la lumière centrale du tube de canule (2) sur une distance comprise de manière préférée entre 0,1 mm et 0,5 mm à partir de la paroi intérieure du tube de canule, est prévue au niveau de la paroi intérieure du tube de canule.

9. Canule trachéale (1) selon la revendication 8, **caractérisée en ce que** la partie de glissement (3b) est réalisée d'un seul tenant avec l'élément profilé (3) et fait saillie dans la lumière centrale du tube de canule (2) à travers une ouverture en forme de fente dudit tube de canule (2) s'étendant sur une partie de la longueur du tube de canule (2).

10. Canule trachéale (1) selon la revendication 8 ou 9, **caractérisée en ce que** la partie de glissement (3b) faisant saillie dans la lumière centrale du tube de canule (2) présente un biseau de glissement (3c).

11. Canule trachéale (1) selon l'une quelconque des revendications 1 à 10, **caractérisée en ce que** le tube de canule (2) et/ou l'élément profilé (3) présente un élément de contraste destiné à un procédé d'imagerie, de manière préférée au diagnostic par rayons X, à la tomographie par résonance magnétique ou à la sonographie.

12. Canule trachéale (1) selon l'une quelconque des revendications 1 à 11, **caractérisée en ce que** l'élément profilé (3) présente un connecteur (3d) permettant le raccordement d'un dispositif de respiration artificielle, dans laquelle l'élément profilé (3) et le connecteur (3c) sont réalisés de manière préférée d'un seul tenant à partir de la même matière plastique.

13. Canule trachéale (1) selon l'une quelconque des revendications 1 à 12, **caractérisée en ce que** le tube de canule (2) présente sur sa surface extérieure un second évidement avec un second élément profilé (3) s'étendant dans la direction axiale, dans laquelle au moins un autre conduit (4) s'étendant de manière axiale est réalisé entre l'élément profilé (3) et le tube de canule (2), dans laquelle le second élément profilé (2) et/ou l'autre conduit (4) sont réalisé(s) selon l'une quelconque des revendications précédentes.

14. Canule trachéale (1) selon l'une quelconque des revendications 1 à 13, **caractérisée en ce que** l'évidement (2e) s'étendant dans la direction axiale présente une saillie, de manière préférée une nervure (2f) s'étendant de manière axiale, et l'élément profilé (3) inséré dans l'évidement présente un évidement (3e) réalisé de manière complémentaire par rapport à la saillie.

15. Canule trachéale (1) selon l'une quelconque des revendications 1 à 14, **caractérisée en ce que** la paroi intérieure et/ou extérieure du tube de canule (2) se rétrécit de manière conique dans la direction axiale entre l'extrémité proximale et l'extrémité distale.

16. Canule trachéale (1) selon l'une quelconque des revendications 1 à 15, **caractérisée en ce que** le tube de canule (2) présente un écran de canule (2g) surmoulé, dans laquelle l'écran de canule (2g) et le tube de canule (2) sont de manière préférée réalisés d'un seul tenant à partir de la même matière plastique.

17. Procédé de fabrication de la canule trachéale (1) selon l'une quelconque des revendications 1 à 16, comprenant les étapes consistant à
- fournir un tube de canule (2) avec une paroi extérieure en retrait le long d'au moins une partie circonférentielle et une épaisseur de paroi réduite de manière correspondante, de sorte qu'un évidement s'étendant dans la direction axiale est défini sur la face extérieure du tube de canule
- fournir le au moins un élément profilé (3),
- agencer le au moins un élément profilé (3) dans l'évidement sur la face extérieure du tube de canule (2) par complémentarité de force et/ou de forme, et
- relier de manière étanche le au moins un élément profilé (3) à la surface extérieure du tube de canule (2) sur la longueur axiale de l'élément profilé.
